# EUROPEAN PATENT APPLICATION

(11) **EP 3 173 421 A1**
(43) Date of publication of application: **31.05.2017**
(21) Application number: 15382594.8
(22) Date of filing: 30.11.2015
(51) Int. Cl.: C07K 7/62, A61K 38/04, C07K 7/06

(54) **PEPTIDIC COMPOUNDS USEFUL AS ANTIBACTERIAL AGENTS**

(71) Applicant: Universitat de Barcelona, 08028 Barcelona (ES)
(72) Inventor: RABANAL ANGLADA, Francesc, 08028 Barcelona (ES)

(57) **Abstract**

It relates to polymyxin analogues having either a short length fatty acyl unit than polymyxin or simply there is a lack of such unit, a hydrophobic chain at the 6^{th} amino acid position of the polymixyn analogue, and in some cases a lower cationic charge than polymyxin, as well as a disulfide bond and an amide or an ester bond in the scaffold. These polymyxin analogues are selective against Gram-negative bacteria and show a particular high antibacterial activity against *P. aeruginosa* resistant strains, thus they are useful in the treatment of bacterial infections. It also relates to pharmaceutical compositions comprising them.

## Description

The present invention relates to compounds which are selective against Gram-negative bacteria, to a preparation process thereof, to pharmaceutical compositions containing them, and to their use in the treatment of bacterial infections.

### BACKGROUND ART

Disease-causing microbes that have become resistant to antibiotic drug therapy are an increasing public health problem. Part of the problem is that bacteria and other microbes that cause infections are remarkably able to develop resistance to antimicrobial drugs. A larger part of the problem is due to the use and misuse of existing antibiotics in human and veterinary medicine, and in agriculture.

Although, it is a significant public health issue in regard to both Gram-positive and Gran-negative bacteria, the problem is most grave for the latter class of bacteria, and more particularly, for *Pseudomonas aeruginosa.* Infections of certain Gram-negative bacteria such as *Pseudomonas aeruginosa* or *Acinetobacter baumannii* are extremely difficult to control, and are prevalent cause of disease and mortality.

The continuous emergence of bacterial strains that are resistant to conventional antibiotics has led to intensive efforts aimed at the development of novel drugs targeting the bacterial cell membranes, such as natural antimicrobial peptides (AMPs). AMPs offer a new class of therapeutic agents to which bacteria may not be able to develop genetic resistance, since they mainly act on the lipid component of the cell membranes. Among these compounds, the clinically approved antibiotic polymyxin B (PxB) is acquiring a new therapeutical relevance and is starting to be considered as a representative of a class of antibiotics which act against multidrug-resistant bacteria.

Polymyxin B is a lipopeptide antibiotic isolated from *Bacillus polymyxa.* Its basic structure consists of a polycationic peptide ring from which is hanging a tripeptide side chain with a fatty acid tail. Polymyxin B has re-emerged in medical practice in recent years and its use will likely continue to increase due to the dry antitbiotic development pipeline and worldwide increasing prevalence of nosocomial infections caused by multidrug-resistant (MDR) Gram-negative bacteria. Polymyxin B and other members of the polymyxin family are drugs of last resort to treat multidrug-resistant infections and sometimes are the only available active antibiotics. In addition, resistance to polymyxins is rare, and generally adaptive and thus reversible. Polymyxin B is also capable of inhibiting the biological activities of bacterial lipopolisaccharide (LPS) through high-affinity binding to the lipid A moiety, being the agent of choice to treat LPS-induced septic shock.

Unfortunately, polymyxins show some nephrotoxicity and neurotoxicity and, therefore, they have limited use. It is known that PxB tends to accumulate over long periods (at least 48h) in kidneys long after administration and after it has become barely detectable in serum (i.e. more than 30 time the serum concentration after 6h). This suggests a selective uptake process in renal cells. Moreover, accumulation of PxB in kidneys could be correlated to its nephrotoxicity potential.

All the attempts of reducing the toxicity by reducing the hydrophobic chain have resulted in loss of activity. T. Velkov et al., in "Structure- Activity Relationships of Polymyxin Antibiotics" J.Med. Chem., 2010, vol.53, pp.1898-1916, discloses several medicinal chemistry strategies assayed with the aim of improving the activity profile of polymyxyns included targeted modifications of the Dab side chains, modification to the cyclic peptide ring and N^{α}fatty acyl chain derivatives. The results suggest that many of the aforementioned modifications generate inactive compounds. In the case of modifications to the N^{α}fatty acyl chain, this document explains that the N-terminal fatty acyl chain is crucial for the antimicrobial activity of polymyxins and that the toxicity of the polymixyin can partly be attributed to this chain. It also explains that the activities of N-terminal fatty acyl derivatives containing C9-C14 unbranched fatty acyl chains showed that the longer fatty acyl derivatives were more active against polymyxin-resistant strains and Gram positive bacteria whereas the C10 and C12 derivatives were most effective against the polymyxin susceptible strains. On the other hand, polymixyn analogues with a short (<C7) unbranched fatty acyl chain displayed an antimicrobial activity lower than polymyxin B.

N. katsuma et al., in "Development of Des-Fatty Acyl-Polymyxin B Decapeptide Analogs with Pseudomonas aeruginosa-Specific Antimicrobial Activity", Chem. Pharm. Bull., 2009, vol. 57, pp. 332-336, discloses several N-terminal analogs of des-fatty acyl-polymyxin B (Des-FA-[X]-PMB). The results indicated that analogs in which X is Lys, Arg, Leu or Ala did not have increased antimicrobial activity against the *E. coli,* S-Typhimurium and P-aeruginosa compared with Des-Fa-[Dab]-PMB and polymyxin B, and Des-FA-[Trp]-PMB and Des-FA-[Phe]-PMB had reduced activity against *P*. *aeruginosa.*

On the other hand, some analogues of polymyxin having a disulfide bond in the skeleton and showing antimicrobial activity against both Gram-negative (*P. aeruginosa* and *E. coli*) and Gram-positive (*S. aureus* and *E. faecalis*) bacteria have been disclosed in WO2010029196, WO2011110716, or WO2014167160.

F. Rabanal et al., in "A bioinspired peptide scaffold with high antibiotic activity and low in vivo toxicity", Scientific Reports, 5, 10558 (2015) explains that the disulfide link provides to the PxB analogs with sufficient stability to reach the infectious target *in vivo* whereas upon eventual accumulation and uptake by renal cells, reduction and subsequent decyclization would facilitate peptide proteolysis and potentially lower renal toxicity. However, the disclosed compounds still have a long N^{α}fatty acyl chain (C₉-C₁₂) which is known to produce certain toxicity but which is needed to achieve a good antibacterial activity. The longer the N^{α}fatty acyl chain, the higher the antibacterial activity, as can be clearly seen from the comparison of the antibacterial activities of compounds 1 and 37 in table 1 of this document, which have the only difference of the length of the fatty acid.

Thus, there is a clear unmet medical need to find high effective antibacterial agents capable of killing pathogens with high selectivity, particularly, multidrug-resistant ones, and which have a low toxicity.

### SUMMARY OF THE INVENTION

Inventors have developed a new line of polymyxin derivatives with reduced toxicity and with high antibacterial activity against Gram-negative bacteria, in particular, against *Pseudomona aeruginosa.*

These compounds are based on the structure of natural polymyxin. In particular, these analogs of polymyxin have either a shorter length fatty acyl unit than polymyxin or simply there is a lack of such unit, and in some cases a lower cationic charge since the basicity of amino groups has also been reduced. These features are known to reduce the toxicity in comparison to polymyxin. Besides, they have an easily metabolizable scaffold thanks to the disulfide bond between the two cysteines and, when appropriate, an ester bond in the macrocycle. The metabolizable scaffold yields a lower toxicity since prevent accumulation in renal cells. The eventual uptake of these analogs by renal cells would presumably reduce the disulfide and open up the peptide cycle due to the reducing intracellular environment (reduced glutathione and oxidorreductases) and thus facilitate the proteolysis of the linear sequence.

The peptidic compounds of the invention are also characterized by having a hydrophobic alkyl chain at the 6^{th} amino acid position of the polymyxin derivative. Thus, with the shortening of the length the fatty acyl unit or the removal of it, and including a hydrophobic alkyl chain at the 6^{th} amino acid position, a redistribution of the hydrophobicity of the molecule is achieved.

Advantageously, the reduction of the toxicity has been achieved without compromising the antibacterial activity. These analogs show good activity against Gram-negative bacteria, and more specifically, a surprising high antibacterial activity against *Pseudomona aeruginosa* particularly resistant strains. Therefore, these compounds show a good selectivity against Gram-negative bacteria and, more in particular, against *P. aeruginosa.*

Furthermore, the compounds are easily prepared by chemical synthesis in good yields.

Thus, an aspect of the present invention is to provide compounds of formula (I), 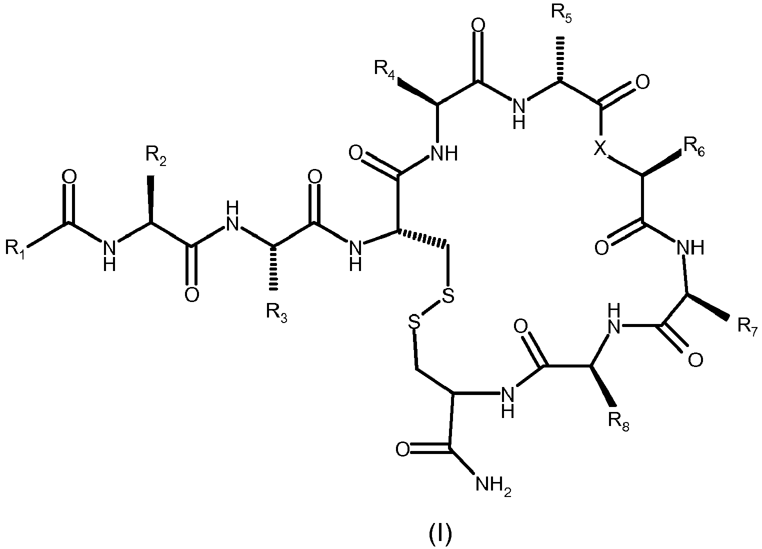 wherein:
R₁ is a radical selected from the group consisting of: 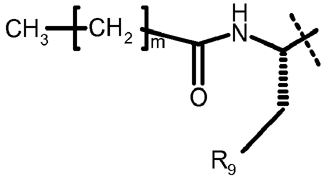 and

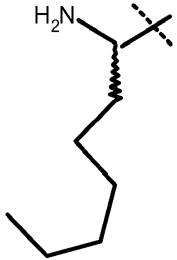

The 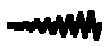 means any of the two configurations D or L; m is an integer from 4 to 6; R₃, R₄, R₇, R₈, and R₉ are independently selected radicals, which have the following formula: GF-(CH₂)ₙ-; n is an integer between 0 and 4; GF is a radical selected from the group consisting of -NH₂, -NH-C(=NH)-NH₂, and imidazolyl; X is NH or O; R₂ is a -CH(CH₃)(OH) radical, where the CH of R₂ is in formula (I) in any of the two configurations R or S, yielding to the corresponding epimers or a mixture thereof; R₅ is a linear or branched (C₄-C₈)-alkyl radical; and R₆ is a linear or branched (C₃-C₆)-alkyl radical.

Another aspect of the present invention relates to the compounds as defined above, for use as an antibacterial agent against Gram-negative bacteria.

Another aspect of the present invention is a pharmaceutical composition comprising a therapeutically effective amount of a compound as defined above, together with appropriate pharmaceutically acceptable excipients or carriers.

Another aspect of the present invention relates to a combination of a therapeutically effective amount of a first antibiotic which is compound as defined above with at least one or more known antibiotics, for use in the treatment of a bacterial infection caused by Gram-negative bacteria, wherein the treatment comprises administering both antibiotics to a subject simultaneously, sequentially or separately.

Finally, another aspect of the present invention relates to a pharmaceutical composition comprising a therapeutically effective amount of a compound of formula (I) as defined above and a therapeutically effective amount of one or more known antibiotics, together with pharmaceutically acceptable excipients or carriers, which is a single dosage form; or alternatively, a kit of parts comprising a pharmaceutical composition comprising a therapeutically effective amount of the compound of formula (I) together with pharmaceutically acceptable excipients or carriers, and one or more pharmaceutical compositions comprising a therapeutically effective amount of a known antibiotic together with pharmaceutically acceptable excipients or carriers.

### DETAILED DESCRIPTION OF THE INVENTION

Preferred compounds of formula (I) are those mentioned above where m is an integer from 4 to 5.

Preferred compounds of formula (I) are those where n is an integer from 1 to 4.

In a particular embodiment, the compounds of formula (I) are those where R₁ is the following:

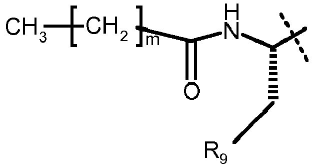

In another particular embodiment, the compounds of formula (I) are those where R₁ is the following:

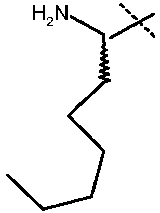

In a preferred embodiment, the compounds of formula (I) are those where when R₁ is this last radical, and the configuration of the amino acid comprising R₁ is D-configuration.

In another particular embodiment, the compounds of formula (I) are those where X is N.

In another particular embodiment, the compounds of formula (I) are those where X is O.

In a preferred embodiment, the compounds of formula (I) are those where R₅ is a linear (C₄-C₈)-alkyl radical. In a more preferred embodiment, the alkyl radical is a linear (C₅-C₇) alkyl radical. In a still more preferred embodiment, the alkyl radical is a linear C₆alkyl radical.

In another particular embodiment, the compounds of formula (I) are those where R₂ is -CH(CH₃)(OH) with the CH in any of the two configurations R or S.

In another particular embodiment, the compounds of formula (I) are those where R₃, R₄, R₇, R₈, and R₉ are a CH₂-CH₂-NH₂ radical.

In another particular embodiment, the compounds of formula (I) are those where R₆ is selected from the group consisting of CH₃-(CH₂)₃-, and (CH₃)₂-(CH)-CH₂-.

In a preferred embodiment, the compounds of formula (I) are selected from the following list:
heptanoyl-Dab-Thr-Dab-cyclo(S-S)[Cys-Dab-DAoc-Nle-Dab-Dab-DCys] (Ia);
heptanoyl-Dab-Thr-Dab-cyclo(S-S)[Cys-Dab-DAoc-OLe-Dab-Dab-DCys] (Ib);
DAoc-Thr-Dab-cyclo(S-S)[Cys-Dab-DAoc-OLe-Dab-Dab-DCys] (Ic);
octanoyl-Dab-Thr-Dab-cyclo(S-S)[Cys-Dab-DAoc-OLe-Dab-Dab-DCys] (Id);
hexanoyl-Dab-Thr-Dab-cyclo(S-S)[Cys-Dab-DAoc-OLe-Dab-Dab-DCys] (le);
heptanoyl-His-Thr-Dab-cyclo(S-S)[Cys-Dab-DAoc-Nle-Dab-His-DCys] (If); and
hexanoyl-Dab-Thr-Dab-cyclo(S-S)[Cys-Dab-DAoc-Nle-Dab-Dab-DCys] (Ig).

The compounds of the present invention can be easily prepared by chemical synthesis; in particular, they can be prepared by Fmoc/tBu solid phase peptide synthesis according to the general methods included in the experimental section.

The compounds obtained can be purified by preparative HPLC with which a final purity greater than 99% can be achieved by this method.

As mentioned above, it is part of the invention the compounds of formula (I) as defined above for use as antibacterial agents against Gram-negative bacteria. Medically relevant Gram-negative bacteria comprise *Escherichia coli, P. aeruginosa and colistin-resistant A. baumannii.*

In a preferred embodiment, the peptidic compounds of the invention are for use as antibacterial agents against multidrug-resistant Gram-negative bacteria. In particular, against multi-drug resistant Gram-negative bacteria such as *Escherichia coli, P. aeruginosa* and *colistin-resistant A. baumannii.*

In a more preferred embodiment, the peptidic compounds of the invention are for use as antibacterial agents against *P. aeruginosa* resistant bacteria.

This aspect of the invention can also be formulated as use of a compound of formula (I) as defined above for the preparation of a medicament for the treatment of a bacterial infection caused by a Gram-negative bacteria, in a mammal, including a human.

The invention also relates to a method of treatment and/or prophylaxis of a mammal, including a human, suffering from or being susceptible to bacterial infection caused by Gram-negative bacteria, in particular to one of the infections mentioned above, the method comprising the administration to the patient of a therapeutically effective amount of the compounds of formula (I) as defined above, together with pharmaceutically acceptable excipients or carriers.

Polymyxin derivatives can disrupt the bacterial outer membrane (OM) and facilitate the access of hydrophobic antibiotics and large hydrophilic antibiotics, such as vancomyin, rifampicin, erythromycin, clarithromycin, tetracyclines, and carbapenems such as imipenem, doripenem and meropenem (cf. M. Vaara, Novel derivatives of polymyxins. J Antimicrob Chemother, 2013 Jun; vol. 68, pp.1213-9; and Wentao Nia et al., In vitro synergy of polymyxins with other antibiotics for Acinetobacter baumannii: A systematic review and meta-analysis, International Journal of Antimicrobial Agents 2015, vol. 45, pp. 8-18.

Compounds of the present invention can be used analogously to other known polymyxin derivatives. They may be used alone or in combination with other suitable bioactive compounds. In a particular embodiment, compounds of formula (I) can be used in the treatment of bacteremia and/or septicemia following infection by Gram-negative bacteria, administered alone or in combination with conventional antibiotics.

Thus, it is part of the invention a combination of a therapeutically effective amount of a first antibiotic which is a compound of formula (I) as defined above and a therapeutically effective amount of one or more known antibiotic, for use in the treatment of a bacterial infection caused by a Gram-negative bacteria, wherein the treatment comprises administering the antibiotics to a subject simultaneously, sequentially or separately.

It is also part of the invention a compound of formula (I) as defined above for use in combination therapy for the treatment of a bacterial infection caused by a Gram-negative bacteria, wherein said medicament is to be administered in combination with one or more known antibiotics.

The known antibiotics can be a hydrophobic antibiotic or a large hydrophilic antibiotic. Examples of suitable antibiotics for the purposes of the invention are vancomyin, rifampicin, erythromycin, clarithromycin, tetracyclines, and carbapenems such as imipenem, doripenem and meropenem.

In a particular embodiment, the compounds of formula (I) of the present invention are for topical or oral use for the decontamination of the digestive tract before surgery.

A pharmaceutical composition comprising a therapeutically effective amount of the compounds of formula (I), together with appropriate amounts of pharmaceutically acceptable excipients or carriers is also part of the invention.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The pharmaceutical compositions may be prepared by combining the compounds of formula (I) of this invention with solid or liquid pharmaceutically acceptable excipients or carriers, in accordance with standard pharmaceutical practices.

The pharmaceutical compositions of this invention may be administered in a manner adequate for the disease that it is desired to treat, for example by oral, parenteral, inhalatory, rectal, transdermal or topical administration. In therapeutic use for treating, or combating bacterial infections in patients such as humans and other animals that can be diagnosed with bacterial infections, the compounds or pharmaceutical compositions thereof, preferably, are administered at a dosage to obtain and maintain a concentration, that is, an amount or blood-level of active component in the patient undergoing treatment which will be antibacterially effective. Generally such antibacterially effective amount of dosage of active component will be in the range of about 0.1 to about 100 mg/Kg, more preferably about 3.0 to about 50 mg/Kg of body weight/day. It is to be understood that the dosages may vary depending upon the requirements of the patient, the severity of the bacterial infection being treated, and the particular compound being used.

Pharmaceutical compositions comprising a therapeutically effective amount of a compound of formula (I) as defined above, and a therapeutically effective amount of one or more known antibiotics, together with pharmaceutically acceptable excipients or carriers, are also part of the invention.

In a particular embodiment, the pharmaceutical composition of the invention comprises a compound of formula (I) and a second antibiotic as active agents. In another particular embodiment, the pharmaceutical composition comprises a compound of formula (I) and two additional antibiotics as active agents.

The active ingredients can be formulated in a single dosage form or independently. When the active ingredients are formulated independently, the respective formulations can form part of a kit of parts. Thus, a kit comprising a pharmaceutical composition comprising a therapeutically effective amount of a compound of formula (I) together with pharmaceutically acceptable excipients or carriers, and one or more pharmaceutical compositions comprising a therapeutically effective amount of a known antibiotic together with pharmaceutically acceptable excipients or carriers, is also part of the invention.

Finally, the present invention covers all the possible combinations of particular and/or preferred embodiments previously mentioned.

Throughout the description and claims the word "comprise" and variations of the word are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention.

### EXAMPLES

Abbreviations: Aoc. 2-aminooctanoic acid; BHA, benzhydrylamine resin; Boc, tert-butoxycarbonyl; Dab: 2,3-diaminobutyric acid; DIEA, N,N-diisopropylethylamine; DIPCDI, N,N'-diisopropylcarbodiimide; DMF, N,N-dimethylformamide; DMSO, dimethylsulfoxide; ESI: electrospray ionization; Fmoc, 9-fluorenylmethoxycarbonyl; HOBt, 1-hydroxybenzotriazole; HPLC,high performance liquid chromatography; MALDI-TOF, matrix-assisted laser desorption ionization time-of-flight; MBHA, 4-methylbenzhydrylamine resin MS, mass spectrometry; MIC, minimum inhibitory concentration; MW, molecular weight; OLe, leucic acid residue; tBu, tert-butyl; TFA, trifluoroacetic acid; and Trt, trityl.

### General methods

To prepare the compounds of the Examples, general protocols of Fmoc/tBu solid phase peptide synthesis were used.

Manual peptide syntheses were performed using polypropylene syringes fitted with a polyethylene disc. The Fmoc-Rink Amide MBHA or BHA resins were used as the solid support.

The Fmoc general synthesis protocol for each amino acid consisted of the following steps: (i) resin washing with DMF (5 x 30 s); (ii) treatment with 20 % piperidine/DMF (1 x 1 min, 2 x 10 min, Fmoc removal); (iii) washing with DMF (5 x 30 s); (iv) acylation with Fmoc-protected amino acid (3 times of excess) with activating reagent DIPCDI/HOBt (3 times of excess for both) in the minimum amount of DMF; (v) washing with DMF (5 x 30 s) and CH₂Cl₂ (5 x 30 s); (vi) Kaiser's test (with a peptide-resin sample); (vii) DMF washing (5 x 30 s).

When a positive result was obtained with Kaiser's test, the Fmoc-protected amino acid (or fatty acid or hydroxy acid) was recoupled with half of the equivalents, i. e. steps (iii) to (vi) were repeated.

When a hydroxy acid was coupled instead of a protected amino acid, step (iv) of the protocol was modified as follows: (iv) acylation with the hydroxy acid (3 times of excess) with activating reagent DIPCDI/HOBt (3 and 6 times of excess, respectively) in the minimum amount of DMF.

The ester bond was obtained in the following cycle by esterification with the corresponding Fmoc-protected amino acid: iv) esterification with the Fmoc-protected amino acid (6 times of excess) using activating reagent DIPCDI (3 times of excess, 25 min preactivation) and then adding DMAP (0.3 equivalents) in the minimum amount of DMF;

The procedure was repeated using additional Fmoc-protected amino acid, DIPCDI and DMAP in proportions (3:1.5:0.15). Completion of the ester bond cannot be monitored with the Kaiser's test in this case, so a pilot cleavage test was carried out with a small peptide-resin sample and monitored by HPLC and ESI-MS.

General cleavage and full deprotection of the peptides was carried out by treatment with trifluoroacetic acid/triisopropylsilane/H₂O (95:3:2, v/v, 90 min + additional 60 min). The TFA solution was filtered off and collected. The resin was rinsed twice with an additional TFA mixture, and the filtrates were combined. The solution was concentrated by means of a stream of nitrogen gas. The oily residue was treated with an excess of dry diethylether to obtain the peptide precipitate. Peptide crudes were washed additionally with ether (twice). The solid peptide was isolated by centrifugation.

Peptide crudes were then dissolved in a 5% DMSO solution in water to a concentration of 1 mM or slightly lower for intramolecular disulfide bond formation. Cyclization reaction was carried out with continuous stirring for 24-36 h. Completion of the cyclization was monitored by HPLC and MS. Peptides were purified routinely by preparative HPLC using a Phenomenex® C18 column (25 x 1 cm, 5 µm diameter) eluting with with 0.1% TFA/H₂O and 0.1% TFA/acetonitrile and UV detection at 220 nm (unless otherwise stated). Mass spectra were obtained in positive mode using an ESI instrument *Spectrometer ZQ-Micromass* (Waters) unless otherwise stated.

By the term "cyclo(S-S)" in the following examples is understood a macrocycle formed by a disulfide bridge between the two cysteines.

### Example 1: Preparation of heptanoyl-Dab-Thr-Dab-cyclo(S-S)[Cys-Dab-DAoc-Nle-Dab-Dab-DCys] (1a), compound of formula I with X = NH, m = 5, R₉ =-CH₂CH₂NH₂ (side chain of Dab), R₂ = -CH(CH₃)OH, (side chain of Thr), R₃ =CH₂CH₂NH₂ (side chain of Dab), R₄ = -CH₂CH₂NH₂ (side chain of Dab), R₅= -(CH₂)₅CH₃, (side chain of DAoc), R₆= -CH₂CH₂CH₂CH₃ (side chain of Nle), R₇ = -CH₂CH₂NH₂ (side chain of Dab), and R₈ = -CH₂CH₂NH₂ (sidechain of Dab)

Protected amino acids: Fmoc-Cys(Trt)-OH, Fmoc-Dab(Boc)-OH, Fmoc-Nle-OH, Fmoc-DAoc-OH, Fmoc-Thr(tBu)-OH, Fmoc-DCys(Trt)-OH.

A manual peptide synthesis was performed following standard Fmoc/^{t}Bu procedures in polypropylene syringes fitted with a polyethylene disc. The Fmoc-Rink Amide MBHA resin (1.5 g, 1.04 mmole, f= 0.69 mmole/g of resin) was used. The amino acids of the sequence were added following a Fmoc/tBu solid phase synthesis protocol using 3 equivalents of excess as described above. Once the full sequence was assembled, heptanoic acid (735 µl, 5.175 mmole, 5 times of excess) was coupled in the presence of HOBt (700 mg, 5.175 mmole, 5 times of excess) and DIPCDI (800 µl, 5.175 mmole, 5 times of excess) in the minimum amount of DMF. The reaction was repeated with 2.588 mmole (2.5 mmole excess) of each of the reagents. After completion of the reaction, the resin was washed with DMF (5 x 30 s) and CH₂Cl₂ (5 x 30 s).

The weight of crude peptide after cleavage and disulfide bond formation was 1.429 g (yield 89%). Purification by HPLC yielded 0.878 g of pure peptide (yield 61 %). Characterization of the purified peptide: Homogeneity (by area integration of HPLC trace) >99%; ESI-MS: m/z 1189.6 ([M+H]⁺, 10%), 595.3 ([M+2H/2]²⁺, 79%), 397.1 ([M+3H/3]³⁺, 88%). MW 1188.68.

### Example 2: Preparation of heptanoyl-Dab-Thr-Dab-cyclo(S-S)[Cys-Dab-DAoc-OLe-Dab-Dab-DCyc] (Ib), (compound of formula I with X = O, m = 5, R₉ = -CH₂CH₂NH₂ (side chain of Dab), R₂ = -CH(CH₃)OH, (side chain of Thr), R₃ = -CH₂CH₂NH₂ (side chain of Dab), R₄ = -CH₂CH₂NH₂ (side chain of Dab), R₅ = -(CH₂)₅CH₃, (side chain of DAoc), R₆= -CH₂CH(CH₃)CH₃(side chain of leucic acid), R₇= -CH₂CH₂NH₂ (side chain of Dab), and R₈= -CH₂CH₂NH₂ (sidechain of Dab)

Protected amino acids: Fmoc-Cys(Trt)-OH, Fmoc-Dab(Boc)-OH, Fmoc-DAoc-OH, Fmoc-Thr(tBu)-OH, Fmoc-DCys(Trt)-OH, leucic acid (unprotected).

A manual peptide synthesis was performed following standard Fmoc/^{t}Bu procedures in polypropylene syringes fitted with a polyethylene disc. The BHA resin (50 mg, 0.0345 mmole, f= 0.69 mmole/g of resin) was used. The Fmoc-Rink linker was incorporated (28 mg, 0.052 mmol, 1.5 eq) to the resin, with DIPCDI (8 microL, 0.052 mmole, 1.5 eq) and HOBt (8 mg, 0.052 mmole, 1.5 eq) and left to react for 12 hours or overnight. A recoupling was performed with 0.5 eq. of the reagents for 2 additional hours. The amino acids of the sequence were added following a Fmoc/tBu solid phase synthesis protocol using 3 equivalents of excess as described above. Once the full sequence was assembled, heptanoic acid (25 µl, 0.17 mmole, 5 times of excess) was coupled in the presence of HOBt (26.4 mg, 0.17 mmole, 5 times of excess) and DIPCDI (26 µl, 0.17 mmole, 5 times of excess) in the minimum amount of DMF.

The weight of crude peptide after cleavage (10 mg, 24 % yield) and disulfide bond formation was 9.7 mg (yield 97%). Purification by HPLC yielded 3 mg of pure peptide (yield 31%). Characterization of the purified peptide: Homogeneity (by area integration of HPLC trace) >99%; ESI-MS: m/z 1190.5 ([M+H⁺]⁺, 100%), 1213.4 ([M+Na]⁺, 9%), MW 1189.67.

### Example 3: Preparation of DAoc-Thr-Dab-cyclo(S-S)[Cys-Dab-DAoc-OLe-Dab-Dab-DCys] (lc), (compound of formula I with X = O, m = does not apply (no fatty acid), R1 = -CH(NH₂)(CH₂)₅CH₃ (moiety of DAoc), R₂ = -CH(CH₃)OH (side chain of Thr), R₃ = -CH₂CH₂NH₂ (side chain of Dab), R₄ = -CH₂CH₂NH₂ (side chain of Dab), R₅= -(CH₂)₅CH₃, (side chain of DAoc), R₆= -CH₂CH(CH₃)CH₃(side chain of leucic acid), R₇= -CH₂CH₂NH₂ (side chain of Dab), and R₈ = -CH₂CH₂NH₂ (sidechain of Dab)

Protected amino acids: Fmoc-Cys(Trt)-OH, Fmoc-Dab(Boc)-OH, Fmoc-DAoc-OH, Fmoc-Thr(tBu)-OH, Fmoc-DCys(Trt)-OH, leucic acid (unprotected).

A manual peptide synthesis was performed following standard Fmoc/^{t}Bu procedures in polypropylene syringes fitted with a polyethylene disc. The BHA resin (56 mg, 0.039 mmole, f= 0.69 mmole/g of resin) was used. The Fmoc-Rink linker was incorporated (32 mg, 0.059 mmol, 1.5 eq) to the resin, with DIPCDI (10 microL, 0.055 mmole, 1.5 eq) and HOBt (10 mg, 0.055 mmole, 1.5 eq) and left to react for 12 hours or overnight. A recoupling was performed with 0.5 eq. of the reagents for 2 additional hours. The amino acids of the sequence were added following a Fmoc/tBu solid phase synthesis protocol using 3 equivalents of excess as described above.

The weight of crude peptide after cleavage (18.5 mg, 43%) and disulfide bond formation was 17.6 mg (yield 95%). Purification by HPLC yielded 3.4 mg of pure peptide (yield 19%). Characterization of the purified peptide: Homogeneity (by area integration of HPLC trace) >99%; ESI-MS: m/z 1119.5 ([M+H]⁺, 12%), 560.3 ([M+2H/2]²⁺, 100%), 373.7 ([M+3H/3]³⁺, 78%). MW 1118.45.

### Example 4: Preparation of octanoyl-Dab-Thr-Dab-cyclo(S-S)[Cys-Dab-DAoc-OLe-Dab-Dab-DCys] (Id), (compound of formula I with X = O, m = 6, R₉ =-CH₂CH₂NH₂ (side chain of Dab), R₂ = -CH(CH₃)OH, (side chain of Thr), R₃ = -CH₂CH₂NH₂ (side chain of Dab), R₄ = -CH₂CH₂NH₂ (side chain of Dab), R₅ = -(CH₂)₅CH₃ (side chain of DAoc), R₆= -CH₂CH(CH₃)CH₃(side chain of leucic acid), R₇= -CH₂CH₂NH₂ (side chain of Dab), and R₈ = -CH₂CH₂NH₂ (sidechain of Dab)

Protected amino acids: Fmoc-Cys(Trt)-OH, Fmoc-Dab(Boc)-OH, Fmoc-DAoc-OH, Fmoc-Thr(tBu)-OH, Fmoc-DCys(Trt)-OH, leucic acid (unprotected).

A manual peptide synthesis was performed following standard Fmoc/^{t}Bu procedures in polypropylene syringes fitted with a polyethylene disc. The BHA resin (53 mg, 0.0366 mmole, f= 0.69 mmole/g of resin) was used. The Fmoc-Rink linker was incorporated (30 mg, 0.055 mmol, 1.5 eq) to the resin, with DIPCDI (9 microL, 0.055 mmole, 1.5 eq) and HOBt (9 mg, 0.055 mmole, 1.5 eq) and left to react for 12 hours or overnight. A recoupling was performed with 0.5 eq. of the reagents for 2 additional hours. The amino acids of the sequence were added following a Fmoc/tBu solid phase synthesis protocol using 3 equivalents of excess as described above. Once the full sequence was assembled, octanoic acid (27 µl, 0.18 mmole, 5 times of excess) was coupled in the presence of HOBt (28 mg, 0.18 mmole, 5 times of excess) and DIPCDI (28 µl, 0.18 mmole, 5 times of excess) in the minimum amount of DMF.

The weight of crude peptide after cleavage (27.3 mg, 62 % yield) and disulfide bond formation was 27.3 mg (yield 100%). Purification by HPLC yielded 6.8 mg of pure peptide (yield 25%). Characterization of the purified peptide: Homogeneity (by area integration of HPLC trace) >99%; ESI-MS: m/z 1204.6 ([M+H⁺], 10%), 602.8 ([M+2H]/2]²⁺ 98%), 402.1 ([M+3H/3]³⁺, 100%). MW 1203.68.

### Example 5: Preparation of hexanoyl-Dab-Thr-Dab-cyclo(S-S)[Cys-Dab-DAoc-OLe-Dab-Dab-DCys] (Ie) (compound of formula I with X = O, m = 4, R₉= -CH₂CH₂NH₂ (side chain of Dab), R₂ = -CH(CH₃)OH, (side chain of Thr), R₃ = -CH₂CH₂NH₂ (side chain of Dab), R₄= -CH₂CH₂NH₂ (side chain of Dab), R₅ = -(CH₂)₅CH₃ (side chain of DAoc), R₆ = -CH₂CH(CH₃)CH₃ (side chain of leucic acid), R₇ = -CH₂CH₂NH₂ (side chain of Dab), and R₈ = -CH₂CH₂NH₂ (sidechain of Dab)

Protected amino acids: Fmoc-Cys(Trt)-OH, Fmoc-Dab(Boc)-OH, Fmoc-DAoc-OH, Fmoc-Thr(tBu)-OH, Fmoc-DCys(Trt)-OH, leucic acid (unprotected).

A manual peptide synthesis was performed following standard Fmoc/^{t}Bu procedures in polypropylene syringes fitted with a polyethylene disc. The BHA resin (46 mg, 0.032 mmole, f= 0.69 mmole/g of resin) was used. The Fmoc-Rink linker was incorporated (26 mg, 0.048 mmol, 1.5 eq) to the resin, with DIPCDI (8 microl, 0.048 mmole, 1.5 eq) and HOBt (8 mg, 0.048 mmole, 1.5 eq) and left to react for 12 hours or overnight. A recoupling was performed with 0.5 eq. of the reagents for 2 additional hours. The amino acids of the sequence were added following a Fmoc/tBu solid phase synthesis protocol using 3 equivalents of excess as described above. Once the full sequence was assembled, hexanoic acid (24 µl, 0.16 mmole, 5 times of excess) was coupled in the presence of HOBt (25 mg, 0.16 mmole, 5 times of excess) and DIPCDI (25 µl, 0.16 mmole, 5 times of excess) in the minimum amount of DMF.

The weight of crude peptide after cleavage was 18.1 mg, (49 % yield) and after disulfide bond formation was 17 mg (yield 94%). Purification by HPLC yielded 6.4 mg of pure peptide (yield 38%). Characterization of the purified peptide: Homogeneity (by area integration of HPLC trace) >99%; ESI-MS: m/z 1176.6 ([M+H]⁺, 4%), 588.7 ([M+2H]/2]²⁺ 46%), 392.70 ([M+3H/3]³⁺, 100%). MW 1176.65

### Example 6: Preparation of heptanoyl-His-Thr-Dab-cyclo(S-S)Cys-Dab-DAoc-Nle-Dab-His-DCys] (If) (compound of formula I with X = O, m = 5, R₉= -CH₂-midazolyl (side chain of His), R₂ = -CH(CH₃)OH, (side chain of Thr), R₃ =CH₂CH₂NH₂ (side chain of Dab), R₄ = -CH₂CH₂NH₂ (side chain of Dab), R₅ = -(CH₂)₅CH₃ (side chain of DAoc), R₆ = -CH₂CH₂CH₂CH₃ (side chain of Nle),R₇ = -CH₂CH₂NH₂ (side chain of Dab), and R₈= -CH₂-imidazolyl (sidechain of His)

Protected amino acids: Fmoc-Cys(Trt)-OH, Fmoc-Cys(Trt)-OH Fmoc-His(Trt)-OH, Fmoc-Nle-OH, Fmoc-DAoc-OH, Fmoc-Thr(tBu)-OH, Fmoc-DCys(Trt)-OH.

A manual peptide synthesis was performed following standard Fmoc/^{t}Bu procedures. The Rink Amide MBHA resin (0.6 g, 1.04 mmole, f= 0.3 mmole/g of resin) was used. The amino acids of the sequence were added following a Fmoc/tBu solid phase synthesis protocol using 3 equivalents of excess as using HBTU/DIEA (3 and 6 times of excess, respectively) as the activating reagent in the minimum amount of DMF. After completion of the reaction, the resin was washed with DMF (5 x 30 s) and CH₂Cl₂ (5 x 30 s). Cleavage and full deprotection was carried out by treatment with trifluoroacetic acid/1,2-ethanedithiol/thioanisole/phenol/H2O /triisopropylsilane (68.5/10/10/5/3.5/1, v/v, 3 ml/100 mg of resin, 3h) in a reaction vessel. The TFA solution was filtered off and collected as described above. The yield of peptide crude was 228 mg (100%).A portion of the peptide crude (50 mg) was then dissolved in a 10% DMSO solution in water to a concentration of ca 0.79 mM for intramolecular disulfide bond formation. Cyclization reaction was carried out with continuous stirring for 36 h. Completion of the cyclization was monitored by HPLC and MS.

Purification was carried out by preparative HPLC. A C18 column (250 x 1 in.,10 µm) eluted with H₂O-acetonitrile-0.05% TFA gradient mixtures and UV detection at 220 nm was used.The weight of crude peptide after cleavage and disulfide bond formation was 50 mg (yield 100%). Purification by HPLC 10 mg of pure peptide (yield 20%).The peptide was characterized by MALDI-TOF mass spectrometry with a Voyager-DE instrument. Homogeneity was assessed by analytical HPLC employing a Kromasil C18 reverse phase column (4'6 x 250 mm, 5 µm of particle diameter). Elution was carried out at 1 ml·min-1 flow with mixtures of H₂O-0.05% TFA and acetonitrile-0.05% TFA and UV detection at 220 nm.

### Example 7: Preparation of hexanoyl-Dab-Thr-Dab-cyclo(S-S)[Cys-Dab-DAoc-Nle-Dab-Dab-DCys], (Ig), compound of formula I with X = NH, m= 4, R₉ = -CH₂CH₂NH₂ (side chain of Dab), R₂ = -CH(CH₃)OH, (side chain of Thr), -CH₂CH₂NH₂ (side chain of Dab), R₄ = -CH₂CH₂NH₂ (side chain of Dab), R₅ = -(CH₂)₅CH₃ (side chain of DAoc), R₆ = -CH₂CH₂CH₂CH₃ (side chain of Nle), R₇ = -CH₂CH₂NH₂ (side chain of Dab), and R₈ = -CH₂CH₂NH₂ (sidechain of Dab)

It was prepared as Example 1, but using hexanoic acid instead of heptanoic acid at a smaller scale. The Fmoc-Rink Amide MBHA resin (67 mg, 0.46 mmole, f= 0.69 mmole/g of resin) was used.

The weight of crude peptide after cleavage and disulfide bond formation was 57.5 mg (yield 100%). Purification by HPLC yielded 11.2 mg of pure peptide (yield 20%).Characterization of the purified peptide: Homogeneity (by area integration of HPLC trace) >99%; MALDI-TOF: m/z 1175.7 ([M+H]⁺, 100%), 1197.7 ([M+Na]⁺, 43%), 1213.7 ([M+K]⁺, 68%). MW 1174.65

### Comparative Example 8: Comparative compounds

Some comparative examples have been included below. Compounds 8a, 8b and 8c corresponds to compounds 1, 37 and 38 published by F Rabanal et al., in "A bioinspired peptide scaffold with high antibiotic activity and low in vivo toxicity", Scientific Reports, 5, 10558 (2015) (see table 1).

These comparative examples have a longer fatty acid chain than the compounds of the invention (C8-C10) and a CH₂-phenyl group in R₅ instead of an alkyl chain.

### Comparative example 8a: Nonanoyl-Dab-Thr-Dab-Cys-Dab-DPhe-Leu-Dab-Dab-Cys, which has the formula:

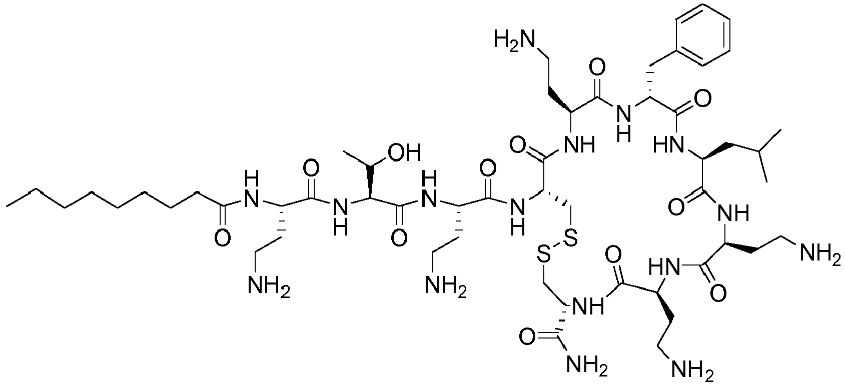

### Comparative example 8b: Decanoyl-Dab-Thr-Dab-Cys-Dab-DPhe-Leu-Dab-Dab-DCys, which has the formula:

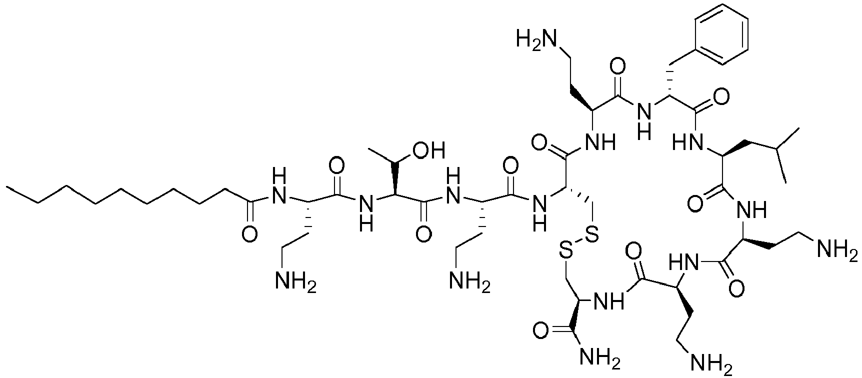

### Comparative example 8c: Decanoyl-Dab-Thr-Dab-Cys-Dab-DPhe-Nle-Dab-Dab-DCys, which has the formula:

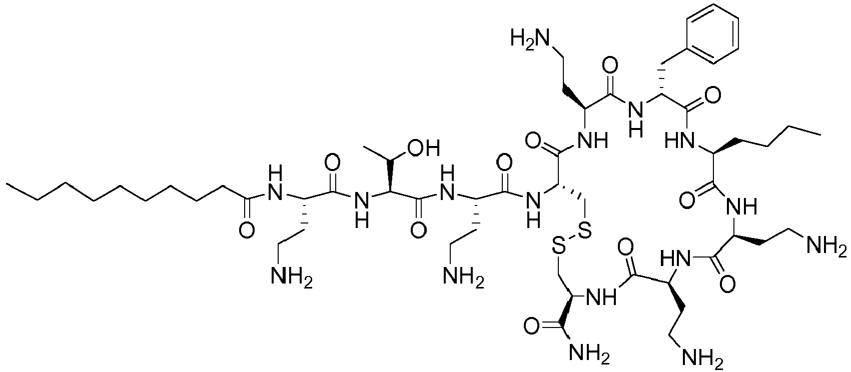

### Comparative example 8d: Polymyxin B

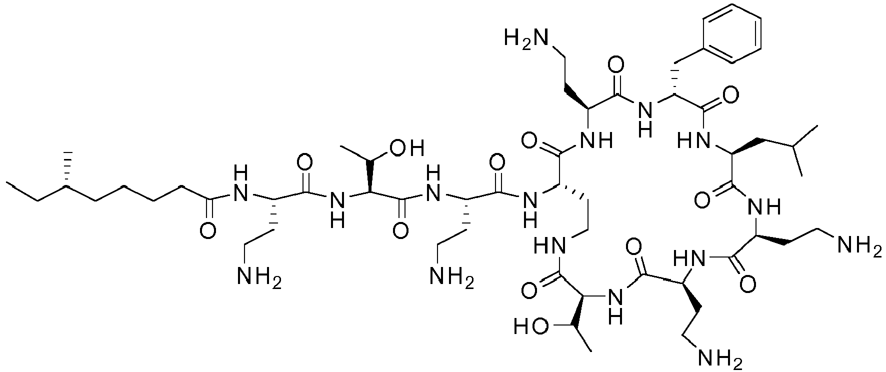

### Polymixyn E colistin

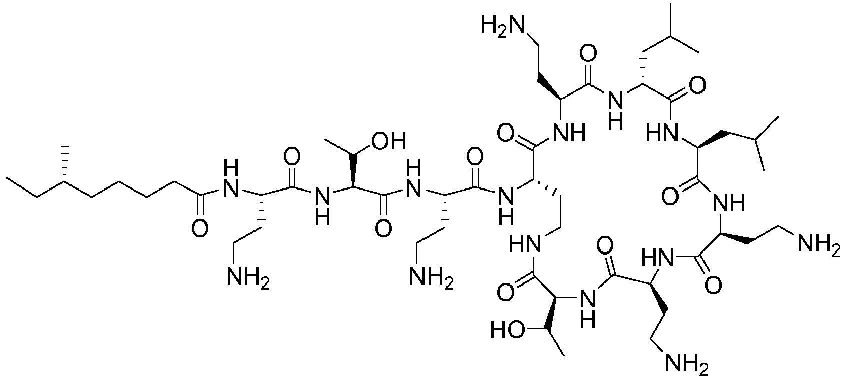

**Table 1: Summary of the compounds**

| Example | Compound | m | R₁ | R₅ |
|---|---|---|---|---|
| Example 1 | C7-Dab-Thr-Dab-Cys-Dab-DAoc-Nle-Dab-Dab-DCys (Ia) | 5 | Heptanoyl | -(CH₂)₅CH₃ |
| Example 2 | C7-Dab-Thr-Dab-Cys-Dab-DAoc-AcLeucic-Dab-Dab-DCys (Ib) | 5 | Heptanoyl | -(CH₂)₅CH₃ |
| Example 3 | DAoc-Thr-Dab-Cys-Dab-DAoc-AcLeucic-Dab-Dab-DCys (Ic) | - | -CH(NH₂)(CH₂)₅CH₃ | -(CH₂)₅CH₃ |
| Example 4 | C8-Dab-Thr-Dab-Cys-Dab-DAoc-AcLeucic-Dab-Dab-DCys (Id) | 6 | Octanoyl | -(CH₂)₅CH₃ |
| Example 5 | C6-Dab-Thr-Dab-Cys-Dab-DAoc-AcLeucic-Dab-Dab-Cys (Ie) | 4 | Hexanoyl | -(CH₂)₅CH₃ |
| Example 6 | C7-His-Thr-Dab-Cys-Dab-DAoc-Nle-Dab-His-DCys (If) | 5 | Heptanoyl | -(CH₂)₅CH₃ |
| Example 7 | C6-Dab-Thr-Dab-Cys-Dab-DAoc-Nle-Dab-Dab-DCys (Ig) | 4 | Hexanoyl | -(CH₂)₅CH₃ |
| Comparative Example 8a | Nonanoyl-Dab-Thr-Dab-Cys-Dab-*D*Phe-Leu-Dab-Dab-Cys | 7 | Nonanoyl | -CH₂-phenyl |
| Comparative Example 8b | Decanoyl-Dab-Thr-Dab-Cys-Dab-DPhe-Leu-Dab-Dab-Cys | 8 | Decanoyl | -CH₂-phenyl |
| Comparative Example 8c | Decanoyl-Dab-Thr-Dab-Cys-Dab-DPhe-Nle-Dab-Dab-Cys | 8 | Decanoyl | -CH₂-phenyl |
| Comparative Example 8d | polymyxin B | 6 (branched) | (S)-6-methyloctanoyl | -CH₂-phenyl |
| Comparative Example 8e | polymyxin E (colistin) | 6 (branched) | (S)-6-methyloctanoyl | CH₂CH(CH₃)₂ |

### Example 9: Antibacterial test for regular and resistant and multi-drug resistant bacteria

The antibacterial activity of the lipopeptides was measured in sterile 96-well plates (Corning Costar 3598 microtiter plates). Samples of a final volume of 200µL were prepared as follows: aliquots (100 µL) of a suspension containing bacteria at a concentration of 10⁵ colony-forming units/mL in culture medium (MH, Muller Hinton Broth, Difco, USA), adjusted at pH 7.4, were added to 100µL of solution containing the peptide prepared from a stock solution of 1 mg/mL peptide in water in serial 2-fold dilutions in MH broth adjusted to pH 7.4 (Jorgensen and Turnide, 2003). Inhibition of bacterial growth was determined by measuring the absorbance at 492 nm with a Absorbance Microplate reader ELx 800 (Bio-tek Instruments) after an incubation of 18-20 h at 37°C. Antibacterial activities are expressed as the MIC, the concentration at which no growth is observed after 18-20 h of incubation. Microorganisms were cultured on Tryptycase Soy Broth (Pronadisa, Barcelona) and incubated at 37°C until growth. Then a loopfull was streaked on Trypticase Soy Agar (Pronadisa, Barcelona) and incubated at 37°C until colony formation occurs. Microorganisms were preserved on cryobilles (EAS laboratoire, France) at -20°C.

Bacterial strains used for the antibacterial activity tests were obtained from the American Type Culture Collection (ATCC, Rockville, MD, USA):
Non resistant Gram-negative bacterias:
   *Escherichia coli* ATCC 8739
   *Pseudomonas aeruginosa* ATCC 9027
Non resistant Gram-positive bacteria:
   *Staphylococcus aureus* ATTC6538

For resistant and multi-drug resistant bacteria Gram negative bacteria, the test was essentially the same with slight changes in the amounts used: samples of a final volume of 100µL were prepared as follows: aliquots (50 *µ*L) of a suspension containing bacteria at a concentration of 1.5x10⁶ colony-forming units/mL in culture medium (MH, Muller Hinton Broth, Difco, USA), adjusted at pH 7.4, were added to 100µL of solution containing the peptide prepared from a stock solution of 1024 mg/L peptide in water in serial 2-fold dilutions in MH broth adjusted to pH 7.4

Samples of resistant and multi-drug resistant bacteria were from clinical origin and not standardized:
Resistant Gram-negative bacterias used:
*Escherichia coli VAL 5*
*Escherichia coli VAL 10*
*Escherichia coli MAC21a*
*Escherichia coli NDM-1*
*Pseudomonas aeruginosa 36a*
*Pseudomonas aeruginosa 38a*
*Pseudomonas aeruginosa 21 R*
*Pseudomonas aeruginosa 21 S*
*Pseudomonas aeruginosa 12R*
*Pseudomonas aeruginosa 12S*
*Colistin-resistant A. baumannii CR49*
*Colistin-resistant A. baumannii Ab99*

*E. coli* strains: NDM-1 (multidrug resistant strain) and VAL10, VAL5 and MAC21 strains show intermediante resistance to quinolones

*P. aeruginosa* strains are highly-resistant: For instance, 38a and 36a are resistant to Ceftazidime, Ciprofloxacin, Imipenem, Piperacillin-tazobactam; 21S is resistant to Ceftazidime, Ciprofloxacin, Imipenem, Piperacillin-tazobactam and tobramycin; 21 R is resistant to Ciprofloxacin, tobramycin and colistin; 12S is resistant to cefotoxin, piperacillin/tazobactam, aztreonam, gentamicin and amikacin; and 12R is resistant to ceftazidime, cefotoxin,cefepime, piperacillin/tazobactam, aztreonam,imipenem, gentamicin, amikacin and colistin.

Resistance Panel for: NDM-1: New Delhi metallo-beta-lactamase: Resistance to (MIC): Amoxicillin 256 mg/L; Amoxicillin clavulanate 32 mg/L ; Piperacillin/tazobactam 256 mg/L; Cefoxitin 256 mg/L; Cefotaxime 256 mg/L; Ceftazidime 256 mg/L; Cefepime 256 mg/L; Imipenem 8 mg/L; Meropenem 16 mg/L; Doripenem 6 mg/L; Ertapenem 24 mg/L; Aztreonam 256 mg/L; Gentamicin 8 mg/L; Amikacin 32 mg/L, Tobramycin 8 mg/L; Ciprofloxacin 32 mg/L (cf. Solé, M. et al. First description of an Escherichia coli strain producing NDM-1 carbapenemase in Spain. Antimicrob. Agents Chemother. 2011, vol. 55, pp. 4402-4404).

The results obtained in the antibacterial test are shown in Tables 2-5. The activities of the comparative examples have been extracted from F Rabanal et al., in "A bioinspired peptide scaffold with high antibiotic activity and low in vivo toxicity", 2015 Scientific Reports, 5, 10558 (2015).

**Table 2: Gram-negative antibacterial activity (MIC) expressed in µg/ml in E. coli**

| | *E coli* | | | | |
|---|---|---|---|---|---|
| Compound | VAL 5 | VAL10 | MAC21 a | NDM-1 | ATCC8739 (non-resistant) |
| Example 1 | 1 | 1 | <0.5 | ≤ 0.5 | 0.5-1 |
| Example 2 | 1 | 32 | 8 | 1 | 4 |
| Example 3 | 2 | 16 | 8 | 2 | 2 |
| Example 4 | 1 | 8 | 8 | 1 | 1 |
| Example 5 | - | - | - | - | 2-4 |
| Example 6 | 8 | 16 | 8 | 8 | 4 |
| Example 7 | - | - | - | - | 8 |
| polymyxin B (8d) | 0.25 | 0.25 | 0.25 | 0.5 | 0.25-0.5 |
| polymyxin E (colistin) (8e) | 0.25 | 0.5 | 0.25 | 0.5 | |
| Comparative 8a | - | - | - | - | 4 |
| Comparative 8b | - | - | - | - | 2 |
| Comparative 8c | 0.5 | 0.5 | 0.5 | 0.5 | 2 |

**Table 3: Gram-negative antibacterial activity (MIC) expressed in µg/ml in P. aeruginosa**

| | P. aeruginosa | | | | | | |
|---|---|---|---|---|---|---|---|
| Compound | 36a | 38a | 21R | 21S | 12R | 12S | ATTC9027 (non-resistant) |
| Example 1 | 0.06 | 0.03 | 0.25 | 0.03 | 1 | 0.5 | 0.5 |
| Example 2 | 1 | 0.125 | 2 | 0.25 | 0.25 | 0.25 | 1-2 |
| Example 3 | 0.5 | 4 | 0.125 | 0.25 | 0.25 | 0.5 | 0.25 |
| Example 4 | 0.125 | 0.125 | 2 | 0.25 | 0.25 | 0.125 | 0.25 |
| Example 5 | - | - | - | - | - | - | <0.25 |
| Example 6 | 1 | 32 | 0.125 | 1 | 2 | 2 | 1 |
| Example 7 | - | - | - | - | - | - | 1-2* |
| polymyxin B (8d) | 2 | 1 | - | - | - | - | 0.25-0.5 |
| polymyxin E (colistin) (8e) | 1 | 1 | - | - | - | - | 1 |
| Comparative Example 8a | - | - | - | - | - | - | 8 |
| Comparative Example 8b | - | - | - | - | - | - | 4 |
| Comparative Example 8c | - | 0.5 | - | - | - | - | 1 |

| | | | | | | | |
|---|---|---|---|---|---|---|---|
| *In the assay where the value of Example 7 was obtained, the polymyxins B gave also a value of 1-2 in the assay of ATTC9027. | | | | | | | |

**Table 4: Gram-negative antibacterial activity (MIC) expressed in µg/ml in colistin-resistant A.baumannii**

| Compound | colistin resistant *A. baumannii* | |
|---|---|---|
| | CR49 | Ab99 |
| Example 2 | 16 | 16 |
| Example 3 | 8 | 8 |
| Example 4 | 16 | 8 |
| Example 6 | 4 | 32 |

**Table 5: Gram-positive antibacterial activity (MIC) expressed in µg/ml**

| Compound | *S*. *aureus* |
|---|---|
| | ATTC6538 |
| Example 1 | 16 |
| Example 2 | 64 |
| Example 5 | 32 |
| Example 6 | 16 |
| Example 7 | >32 |
| polymyxin B | >32 |
| Comparative Example 8a | >32 |
| Comparative Example 8b | 8 |
| Comparative Example 8c | 4 |

The previous results prove that the compounds of the invention (analogues of polymyxin having either a shorter length or a lack of fatty acyl unit, and lower cationic charge as well as a hydrophobic chain at the 6^{th} amino acid position) are selective against Gram negative bacteria in front of Gram positive bacteria and are active against non-resistant and resistant-strains. In particular, they are very active against P. aeruginosa particularly resistant strains.

Thus, the reduction of the toxicity due to the N^{α}fatty acyl chain, has been achieved without compromising the activity. Surprisingly, they have resulted to be extremely active against *P. aeruginosa* particularly resistant strain.

### REFERENCES CITED IN THE APPLICATION

- WO2010029196
- WO2011110716
- WO2014167160
- F Rabanal et al., "A bioinspired peptide scaffold with high antibiotic activity and low in vivo toxicity", Scientific Reports, 5, 10558 (2015) (www.nature.com/scientificreports 5:10558*)*
- T. Velkov et al., "Structure- Activity Relationships of Polymyxin Antibiotics" J.Med: Chem., 2010, vol.53, pp.1898-1916
- N. katsuma et al., " Development of Des-Fatty Acyl-Polymyxin B Decapeptide Analogs with Pseudomonas aeruginosa-Specific Antimicrobial Activity", Chem. Pharm. Bull. 2009, vol. 57, pp. 332-336
- Solé, M. et al. First description of an Escherichia coli strain producing NDM-1 carbapenemase in Spain. Antimicrob. Agents Chemother. 2011, vol. 55, pp. 4402-4404
- M. Vaara, Novel derivatives of polymyxins. J Antimicrob Chemother, 2013 Jun, vol. 68, pp.1213-9
- Wentao Nia et al., In vitro synergy of polymyxins with other antibiotics for Acinetobacter baumannii: A systematic review and meta-analysis, International Journal of Antimicrobial Agents 2015, vol. 45, pp. 8-18.

## Claims

1. A compound of formula (I), 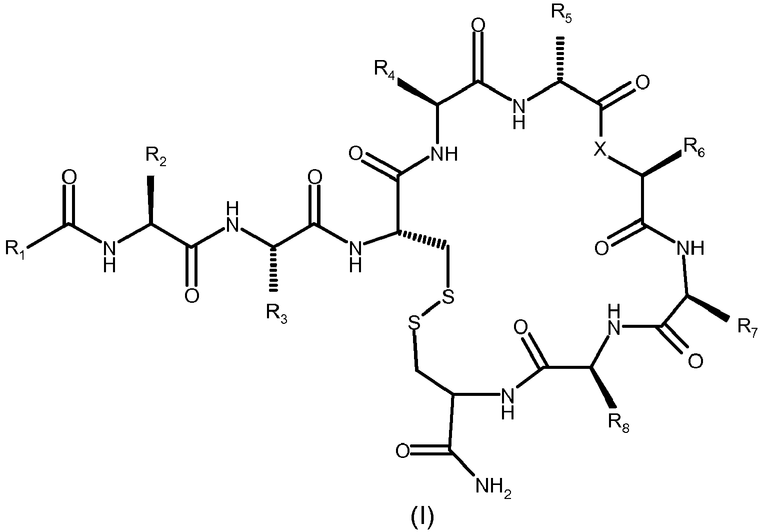 wherein:
R₁ is a radical selected from the group consisting of: 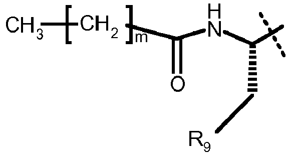 and 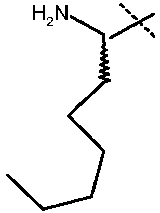
The 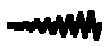 means any of the two configurations D or L;
m is an integer from 4 to 6;
R₃, R₄, R₇, R₈, and R₉ are radicals independently selected having the following formula:
GF-(CH₂)ₙ-;
n is an integer between 0 and 4;
GF is a radical selected from the group consisting of -NH₂, -NH-C(=NH)-NH₂, and imidazolyl;
X is NH or O;
R₂ is a -CH(CH₃)(OH) radical where the CH of R₂ is in formula (I) in any of the two configurations R or S, yielding to the corresponding epimers or a mixture thereof;
R₅ is a linear or branched (C₄-C₈)-alkyl radical; and
R₆ is a linear or branched (C₃-C₆)-alkyl radical.

2. The compound according to claim 1, wherein m is an integer from 4 to 5.

3. The compound according to any of the claims 1-2, wherein R₁ is the following:

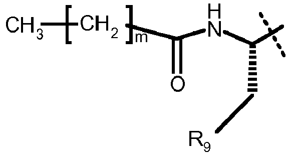

4. The compound according to any of the claims 1-2, wherein R₁ is the following:

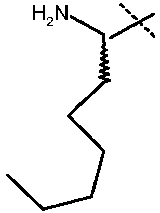

5. The compound according to claim 4, wherein the configuration of the amino acid comprising R₁ is D-configuration.

6. The compound according to any of the claims 1-5, wherein X is N.

7. The compound according to any of the claims 1-6, wherein R₅ is a linear C₆ alkyl radical.

8. The compound according to any of the claims 1-7, wherein R₃, R₄, R₇, R₈ and R₉ are a CH₂CH₂-NH₂ radical.

9. The compound according to any of the claims 1-8, wherein R₆ is selected from the group consisting of CH₃-(CH₂)₃- and (CH₃)₂-(CH)-CH₂-.

10. The compound according to claim 1, which is selected from the group consisting of:
heptanoyl-Dab-Thr-Dab-cyclo(S-S)[Cys-Dab-DAoc-Nle-Dab-Dab-DCys] (Ia);
heptanoyl-Dab-Thr-Dab-cyclo(S-S)[Cys-Dab-DAoc-OLe-Dab-Dab-DCys] (Ib);
DAoc-Thr-Dab-cyclo(S-S)[Cys-Dab-DAoc-OLe-Dab-Dab-DCys] (Ic);
octanoyl-Dab-Thr-Dab-cyclo(S-S)[Cys-Dab-DAoc-OLe-Dab-Dab-DCys] (Id);
hexanoyl-Dab-Thr-Dab-cyclo(S-S)[Cys-Dab-DAoc-OLe-Dab-Dab-DCys] (Ie);
heptanoyl-His-Thr-Dab-cyclo(S-S)[Cys-Dab-DAoc-Nle-Dab-His-DCys] (If); and
hexanoyl-Dab-Thr-Dab-cyclo(S-S)[Cys-Dab-DAoc-Nle-Dab-Dab-DCys] (Ig).

11. A compound as defined in any of the claims 1-10, for use as antibacterial agent against Gram-negative bacteria.

12. The compound according to claim 11, wherein the Gram-negative bacteria is selected from the group consisting of *A. baumannii, Pseudomonas aeruginosa,* and *Escherichia coli.*

13. A pharmaceutical composition comprising a therapeutically effective amount of a compound as defined in any of the claims 1-10, together with appropriate pharmaceutically acceptable excipients or carriers.

14. A combination of a therapeutically effective amount of a compound of formula (I) as defined in any of the claims 1-10 and a therapeutically effective amount of one or more known antibiotics, for use in the treatment of a bacterial infection caused by Gram-negative bacteria, wherein the treatment comprises administering the antibiotics to a subject simultaneously, sequentially or separately.

15. A pharmaceutical composition comprising a therapeutically effective amount of a compound of formula (I) as defined in any of the claims 1-10 and a therapeutically effective amount of one or more known antibiotics, together with pharmaceutically acceptable excipients or carriers, which is a single dosage form; or alternatively, a kit of parts comprising a pharmaceutical composition comprising a therapeutically effective amount of the compound of formula (I) together with pharmaceutically acceptable excipients or carriers, and one or more pharmaceutical compositions comprising a therapeutically effective amount of a known antibiotic together with pharmaceutically acceptable excipients or carriers.
